# EUROPÄISCHE PATENTSCHRIFT

(11) **EP 1 099 682 B1**
(45) Veröffentlichungstag und Bekanntmachung des Hinweises auf die Patenterteilung: **04.06.2003**
(21) Anmeldenummer: 00123373.3
(22) Anmeldetag: 31.10.2000
(51) Int. Cl.: C07C 201/00, C07C 207/02, C07C 207/04

(54) **Verfahren zur Herstellung von Nitrosobenzolen**
Process for the preparation of nitrosobenzenes
Procédé pour la préparation de nitrosobenzènes

(30) Priorität: 12.11.1999 DE 19954397
(43) Veröffentlichungstag der Anmeldung: 16.05.2001
(73) Patentinhaber: Bayer Aktiengesellschaft, 51368 Leverkusen (DE)
(72) Erfinder: Giera, Henry, Dr., 86862 Grosskitzighofen (DE); Meiers, Michaela, Dr., 67346 Speyer (DE); Hugger, Uwe, Dr., 25462 Rellingen (DE)

(56) Entgegenhaltungen:
- DATABASE WPI Section Ch, Week 199619 Derwent Publications Ltd., London, GB; Class A60, AN 1996-186751 XP002161861 & RU 2 042 661 C (KRASY TECHN ACAD), 27. August 1995 (1995-08-27)
- TOLLARI, STEFANO ET AL: "Catalytic oxidation of primary aromatic amines to the corresponding nitroso compounds by H2O2 and [Mo(O)(O2)2(H2O)(hmpa)] (hmpa = hexamethylphosphoric triamide)" J. CHEM. SOC., CHEM. COMMUN. (1993), (19), 1510-11 , XP002161859
- SAKAUE, SHIGEKI ET AL: "Oxidation of aliphatic and aromatic amines with hydrogen peroxide catalyzed by peroxoheteropoly oxometalates" CHEM. LETT. (1992), (2), 289-92 , XP002161860
- PORTA, F. ET AL: "Catalytic synthesis of C-nitroso compounds by cis-Mo(O)2(acac)2" J. MOL. CATAL. A: CHEM. (2000), 157(1-2), 123-129 , XP000982547

## Beschreibung

Die Erfindung betrifft ein Verfahren zur Herstellung von Nitrosobenzolen aus aromatischen Aminen durch Oxidation mit Wasserstoffperoxid in Gegenwart eines Katalysator ohne den Zusatz der üblicherweise verwendeten organischen Lösungsmittel.

Die Herstellung von Nitrosobenzol, bei dem es sich um ein wichtiges Zwischenprodukt handelt, ist bekannt und kann nach verschiedenen Methoden durchgeführt werden. Es kann zur Synthese von Alterungsschutzmitteln und Stabilisatoren in der Gummi- und Polymerindustrie, insbesondere zur Herstellung von 4-Aminodiphenylamin (4-ADPA) eingesetzt werden.

Eine Methode zur Herstellung von Nitrosobenzolen ist die Oxidation von entsprechenden aromatischen Aminen mit geeigneten Oxidationsmitteln, wie Wasserstoffperoxid, in Anwesenheit eines Katalysators sowie in Gegenwart eines geeigneten Lösungsmittels.

Die Herstellung von Nitrosobenzolen aus aromatischen Aminen durch katalytische Oxidation mit Wasserstoffperoxid ist beispielsweise beschrieben von S. Sakaue, T. Tsubakino, Y. Nishiyama, Y. Ishii. *Org. Chem.* **1993**, 58, 3633; S. Tollari, M. Cuscela, F. Porta, *J. chem. Soc., Chem. Commun.* **1993**, 1510 sowie in RU-A 1680689; RU-A 2042661; RU-A 2044724; RU-A 2090542 und von E.B. Mel'nikov, G.A. Suboch, E. Yu. Belyaev, *Russ. J. Org. Chem.* **1995**, 31, 160-1642 sowie Z. Zhu, J.H. Espensen, *J. Org. Chem.* **1995**, 60, 1326-1332.

Nachteile bei den angegebenen Verfahren sind z.B. der Einsatz großtechnisch nicht praktikabler Mengen risikobelasteter Lösungsmittel, wie Chloroform, um gute Ausbeuten zu erzielen, die Verwendung von toxischen Verbindungen, wie Hexamethylphosphorsäuretriamid, bei der Katalysatorherstellung, die Verwendung von zusätzlichen Co-Katalysatoren und der Einsatz größerer Katalysatormengen sowie die Verwendung von teuren Katalysatoren. Durch die gegebenen Nachteile würde sich ein großtechnisch betriebenes Verfahren unwirtschaftlich gestalten verbunden mit zum Teil umfangreichen Sicherheitsmaßnahmen.

Es wurde nun gefunden, dass man Nitrosobenzole aus aromatischen Aminen durch katalytische Oxidation mit Wasserstoffperoxiden in technisch einfacher Weise und unter Vermeidung der oben geschilderten Nachteile herstellen kann, wenn man die Oxidation ohne den Zusatz von Lösungsmittel durchführt.

Gegenstand der vorliegenden Erfindung ist daher ein Verfahren zur Herstellung von Nitrosobenzolen der allgemeinen Formel 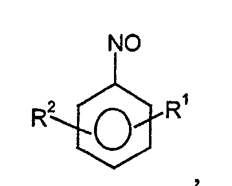 worin
R¹ und R² gleich oder verschieden sind und für Wasserstoff, C₁-C₄-Alkyl oder C₁-C₄-Alkoxy stehen,
durch Oxidation entsprechender aromatischer Amine, das dadurch gekennzeichnet ist, dass man die Oxidation mit Wasserstoffperoxid in Gegenwart eines Oxidationskatalysators auf Basis von Wolfram- und/oder Molybdän-Verbindungen bei Temperaturen von 5 bis 25°C in Abwesenheit eines organischen Lösungsmittels bei einem Molverhältnis von aromatischen Aminen zu Wasserstoffperoxid zu Oxidationskatalysator von 1:2,0 bis 5,0:0,001 bis 0,05 durchführt. Bevorzugt eingesetzt werden aromatische Amine, wie z.B. Anilin, o-, m- und p-Toluidin, o-, m-, p-Anisidin, ganz besonders bevorzugt Anilin.

Bevorzugt werden Nitrosobenzole der obigen Formel hergestellt, in denen R¹ und R² für Wasserstoff, Methyl- oder Methoxy-Gruppen stehen. Ganz besonders bevorzugt ist die Herstellung von Nitrosobenzol (d.h. R¹ und R² = Wasserstoff).

Das erfindungsgemäße Verfahren wird bevorzugt bei Temperaturen von 15 bis 25°C durchgeführt.

Das erfindungsgemäß eingesetzte Wasserstoffperoxid wird üblicherweise in wässriger Form in einer Konzentration von 15 bis 80, bevorzugt 30 bis 50 Gew.-% Wasserstoffperoxid verwendet.

Bevorzugt wird das erfindungsgemäße Verfahren bei einem Molverhältnis von aromatischen Aminen zu Wasserstoffperoxid zu Oxidationskatalysator von 1:2,5 bis 4,5:0,003 bis 0,02 durchgeführt.

Als erfindungsgemäß einzusetzende Oxidationskatalysatoren kommen die aus der entsprechenden Literatur bekannten Oxidationskatalysatoren auf Basis von Wolframund Molybdän-Verbindungen in Betracht. Bevorzugt zu nennen sind Mo- und W-Oxide, Molybdän- und Wolframsäuren, Salze der Molybdän- und Wolframsäuren, Salze der Polymolybdän- und Polywolframsäuren, Molybdän- und Wolfram-enthaltende Polyoxometallate, Molybdän- und Wolfram-enthaltende Heteropolysäuren und deren Salze sowie Molybdän- und Wolfram-oxo-Komplexe.

Ganz besonders bevorzugt sind die quaternären Ammoniumsalze, die Alkali- und Erdalkalimetallsalze der oben genannten Säuren sowie Molybdän und Wolfram-enthaltende Heteropolysäuren und/oder Molybdän- und Wolfram-oxo-Komplexe.

Insbesondere sind im einzelnen zu nennen Wolframatophosphorsäure, Peroxocetylpyridiniummolybdophosphat (PCMP), Tetrakistetrabutylammoniumoctamolybdat, Cetylpyridiniumwolframatophosphat (CWP), Cetylpyridiniummolybdatophosphat (CMP), Molybdändioxidbisacetylacetonat, Natriummolybdat und/oder Natriumwolframat.

Die einzusetzenden Katalysatoren können sowohl einzeln als auch im Gemisch untereinander eingesetzt werden. Das günstigste Mischungsverhältnis ist leicht durch entsprechende Vorversuche zu bestimmen und hängt u.a. von den angewendeten Reaktionsbedingungen ab.

Nach Beendigung des erfindungsgemäßen Verfahrens kann das erhaltene Nitrosobenzol in üblicherweise aus der Reaktionsmischung isoliert werden, durch z.B. Abfiltrieren des ausgefallenen Feststoffes, Waschen des Feststoffes mit Wasser und anschließendem Trocknen.

Die Nitrosobenzole werden in Ausbeuten von 50 bis 95 % der Theorie und in Reinheiten von ≥ 95 % erhalten.

Bei dem erfindungsgemäßen Verfahren überrascht besonders, dass es - im Gegensatz zu dem bisherigen Stand der Technik - ohne den Zusatz von Lösungsmittel durchgeführt werden kann, ohne das dabei Ausbeuteverluste oder Einbußen bei der Reinheit hingenommen werden müssen. Da auf die Verwendung von zusätzlichen Lösungsmittel verzichtet werden kann bei dem erfmdungsgemäßen Verfahren, gestaltet sich dieses besonders wirtschaftlich.

### Beispiele

### Allgemeine Arbeitsvorschrift:

Der Katalysator wird vorgelegt, 100 µl (1,10 mmol) Anilin werden zugesetzt und mit 350 µl (3,4 mmol) Wasserstoffperoxid versetzt. Die Reaktionstemperatur beträgt 20°C. Nach 2 h wird die Reaktion beendet und die Ausbeute wird mit einer GC-Methode bestimmt gemäß
1.) Vogel's, Textbook of Quantitative Chemical Analysis, 5^{th} Edition, Longwan Scientific & Technical, S. 247
2.) H. Hulpke, H. Hartkamp, G. Tölg (Hrsg.), Analytische Chemie für die Praxis, K. Beyermann, Organische Spurenanalyse, G. Thieme Verlag, Stuttgart, New York, 1982, S. 37-41.

| Beispiel Nr. | Katalysator | Katalysatormenge (Äq.) | Ausbeute (%) |
|---|---|---|---|
| 1 | H₃PW₁₂O₄₀ | 0,003 | 51,0 |
| 2 | PCMP | 0,003 | 90,2 |
| 3 | [(n-C₄H₉)₄N]₄[Mo₈O₂₆] | 0,004 | 94,4 |
| 4 | CWP | 0,003 | 61,0 |
| 5 | CMP | 0,003 | 90,8 |
| 6 | MoO₂(acac)₂ | 0,02 | 80,6 |
| 7 | Na₂MoO₄·2H₂O | 0,02 | 71,0 |

### Beispiel 8

Zu einer Mischung von 22,5 g (0,068 mol) Natriumwolframat und 1457 g (12,9 mol) Wasserstoffperoxid werden bei 20°C 465 g (5 mol) Anilin getropft. Zunächst färbt sich die Reaktionsmischung gelb-grün und ein beiger Feststoff fällt aus. Nach erfolgter Anilinzugabe wird der Feststoff abfiltriert, mehrfach mit insgesamt 500 ml Wasser gewaschen und über Calciumchlorid getrocknet. Man erhält 485 g (91 % der Theorie) eines beige-braunen Feststoffs mit einem Gehalt von 98 % Nitrosobenzol.

## Patentansprüche

1. Verfahren zur Herstellung von Nitrosobenzolen der allgemeinen Formel 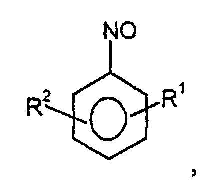 worin
R¹ und R² gleich oder verschieden sind und für Wasserstoff, C₁-C₄-Alkyl oder C₁-C₄-Alkoxy stehen,
durch Oxidation entsprechender aromatischer Amine, **dadurch gekennzeichnet, dass** man die Oxidation mit Wasserstoffperoxid in Gegenwart eines Oxidationskatalysators auf Basis von Wolfram- und/oder Molybdän-Verbindungen bei Temperaturen von 5 bis 25°C in Abwesenheit eines organischen Lösungsmittels bei einem Molverhältnis von aromatischen Aminen zu Wasserstoffperoxid zu Oxidationskatalysator von 1:2,0 bis 5,0:0,001 bis 0,05 durchführt.

2. Verfahren zur Herstellung von Nitrosobenzol nach Anspruch 1, **dadurch gekennzeichnet, dass** bei einem Molverhältnis von aromatischen Aminen zu Wasserstoffperoxid zu Oxidationskatalysator von 1:2,5 bis 4,5:0,003 bis 0,02 gearbeitet wird.

3. Verfahren zur Herstellung von Nitrosobenzol nach Anspruch 1, **dadurch gekennzeichnet, dass** man als Wolfram- und/oder Molybdän-Verbindungen die Mo- und W-Oxide, Molybdän- und Wolframsäuren, Salze der Molybdän- und Wolframsäure, Salze der Polymolybdän- und Polywolframsäuren, Molybdänund Wolfram-enthaltende Polyoxometallate, Molybdän- und Wolfram-enthaltende Heteropolysäuren und deren Salze und/oder Molybdän- und Wolfram-oxo-Komplexe einsetzt.

## Claims

1. Process for the preparation of nitrosobenzenes of the general formula 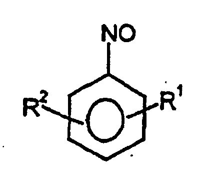 wherein
R¹ and R² are identical or different and represent hydrogen, C₁-C₄-alkyl or C₁-C₄-alkoxy, by oxidation of corresponding aromatic amines, **characterized in that** the oxidation is carried out with hydrogen peroxide in the presence of an oxidation catalyst based on tungsten compounds and/or molybdenum compounds at temperatures of 5 to 25°C in the absence of an organic solvent at a molar ratio of aromatic amines to hydrogen peroxide to oxidation catalyst of 1:2.0 to 5.0:0.001 to 0.05.

2. Process for the preparation of nitrosobenzene according to claim 1, **characterized in that** it is carried out at a molar ratio of aromatic amines to hydrogen peroxide to oxidation catalyst of 1:2.5 to 4.5:0.003 to 0.02.

3. Process for the preparation of nitrosobenzene according to claim 1, **characterized in that** the Mo oxides and W oxides, molybdic and tungstic acids, salts of molybdic and tungstic acid, salts of polymolybdic and polytungstic acids, molybdenum- and tungsten-containing polyoxometallates, molybdenum- and tungsten-containing heteropolyacids and salts thereof and/or molybdenum- and tungsten-oxo complexes are employed as the tungsten compounds and/or molybdenum compounds.

## Revendications

1. Procédé de préparation de nitrosobenzènes de la formule générale : 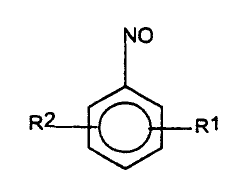 où R¹ et R² sont identiques ou différents et représentent l'atome d'hydrogène, un radical alcoyle en C₁-C₄ ou alcoxy en C₁-C₄,
par oxydation de l'amine aromatique correspondante, qui est **caractérisé en ce que** l'on réalise l'oxydation avec le peroxyde d'hydrogène en présence d'un catalyseur d'oxydation à base de composés du tungstène et/ou du molybdène à des températures allant de 5 à 25°C, en l'absence de solvant organique, à un rapport molaire des amines aromatiques au peroxyde d'hydrogène au catalyseur d'oxydation de 1:2,0 à 5,0:0,001 à 0,05.

2. Procédé de préparation de nitrosobenzènes selon la revendication 1, **caractérisé en ce que** l'on travaille à un rapport molaire des amines aromatiques au peroxyde d'hydrogène au catalyseur d'oxydation allant de 1:2,5 à 4,5:0,003 à 0,02.

3. Procédé de préparation de nitrosobenzènes selon la revendication 1, **caractérisé en ce que** l'on met en oeuvre comme composés du tungstène et/ou du molybdène, les oxydes de Mo et/ou de W, les acides de molybdène et de tungstène, les sels d'acides de molybdène et de tungstène, les sels de polyacides de molybdène et de tungstène, les polyoxométallates contenant du molybdène et du tungstène, les hétéropolyacides contenant du molybdène et du tungstène et leurs sels ainsi que des oxocomplexes du molybdène et du tungstène.
